(19) Europäisches Patentamt
European Patent Office
Office européen des brevets

(11) Veröffentlichungsnummer: **0 001 798**
**A1**

(12) **EUROPÄISCHE PATENTANMELDUNG**

(21) Anmeldenummer: **78101207.5**

(22) Anmeldetag: **24.10.78**

(51) Int. Cl.$^2$: **C 07 C 85/20**

(30) Priorität: **04.11.77 DE 2749415**

(43) Veröffentlichungstag der Anmeldung:
**16.05.79 Patentblatt 79/10**

(84) Benannte Vertragsstaaten:
**BE CH DE FR GB NL**

(71) Anmelder: **Bayer Aktiengesellschaft**
**Zentralbereich Patente,Marken und Lizenzen Bayerwerk**
**D-5090 Leverkusen 1(DE)**

(72) Erfinder: **Arlt, Dieter, Dr.**
**Rybnikerstrasse 2**
**D-5000 Köln 80(DE)**

(54) **Verfahren zur Herstellung von Aminen aus Formamiden.**

(57) Die Erfindung betrifft ein Verfahren zur Herstellung von Aminen aus Formamiden, bei dem ein Formamid mit einem Amin bei Temperaturen im Bereich von 75 bis 250°C umsetzt und das aus dem eingesetzten Formamid abgespaltene Amin und das aus dem eingesetzten Amin neu gebildete Formamid destillativ auftrennt.

EP 0 001 798 A1

Croydon Printing Company Ltd.

BAYER AKTIENGESELLSCHAFT
Zentralbereich
Patente, Marken und Lizenzen

5090 Leverkusen, Bayerwerk
Ha/bc

Verfahren zur Herstellung von Aminen aus Formamiden

Die vorliegende Erfindung betrifft ein Verfahren zur Herstellung von Aminen aus Formamiden.

Aus der DT-PS 870 856 ist ein Verfahren zur Herstellung von Formamiden bzw. Aminen bekannt, bei dem man Blausäure in Gegenwart von stark sauren Kondensationsmitteln auf Kohlenwasserstoffe mit einer olefinischen Doppelbindung einwirken läßt und die erhaltenen Additionsprodukte zu den entsprechenden Formamiden hydrolysiert und gegebenenfalls die so erhaltenen Formamide zu den entsprechenden Aminen verseift.

Weiter ist bekannt (DT-AS 1 059 468), wasserfreie Formamide mit festem, trockenem Alkalihydroxid bei erhöhten Temperaturen umzusetzen, wobei Alkaliformiat und Amin gebildet werden.

Es wurde ein Verfahren zur Herstellung von Aminen aus Formamiden gefunden, daß dadurch gekennzeichnet ist, daß man ein Formamid der Formel

BAD ORIGINAL

$R^1$-NH-CHO (I),

in der

$R^1$ Alkyl, Cycloalkyl oder Aralkyl bedeutet,

mit einem Amin der Formel

$R^2R^3NH$ (II),

in der

$R^2$ und $R^3$ Wasserstoff, Alkyl, Cycloalkyl, Aralkyl oder Aryl bedeuten,
oder in der die Reste $R^2$ und $R^3$ gemeinsam mit dem Stickstoffatom und gegebenenfalls weiteren Heteroatomen einen 5- bis 8-gliedrigen heterocyclischen Ring bilden,
wobei nur einer der Reste $R^2$ und $R^3$ Wasserstoff sein kann und
wobei wenigstens einer der Reste $R^2$ und $R^3$ verschieden von $R^1$ ist,

bei erhöhter Temperatur umsetzt und den niedriger siedenden Anteil der Umsetzungsprodukte destillativ aus dem Reaktionsgemisch entfernt.

Als Alkylreste ($R^1$) seien geradkettige oder verzweigte Kohlenwasserstoffreste mit 1 bis 16, bevorzugt 1 bis 12, besonders bevorzugt 1 bis 6, Kohlenstoffatomen genannt, beispielsweise Methyl, Äthyl, Propyl, Isopropyl, Butyl, Isobutyl, tert.-Butyl, Hexyl, Octyl, Dodecyl, Hexadecyl.

Bevorzugte Alkylreste ($R^1$) sind solche, die in $\alpha$-Stellung verzweigt sind, beispielsweise Isopropyl, tert.-Butyl, sek.-Butyl, Isohexyl, Isooctyl, Isododecyl und Isohexadecyl.

BAD ORIGINAL

Insbesondere wird als Alkylrest ($R^1$) der tert.-Butylrest bevorzugt.

Als Cycloalkylreste ($R^1$) seien solche mit 5 bis 8 Ringgliedern, beispielsweise Cyclopentyl, Methyl-Cyclopentyl, Cyclohexyl, Methylcyclohexyl, Cycloheptyl oder Cyclooctyl, genannt.

Als Aralkylreste ($R^1$) seien solche mit 1 bis 4 Kohlenstoffatomen im aliphatischen Teil und 6 bis 12 Kohlenstoffatomen im aromatischen Teil, beispielsweise Benzyl, ß-Phenyl-äthyl, Naphthyl-methyl oder ß-Naphthyl-äthyl, genannt.

Bevorzugte Aralkylreste ($R^1$) sind solche, die in α-Stellung verzweigt sind, beispielsweise α-Phenyl-äthyl oder Benzhydryl.

Alkylreste ($R^2$,$R^3$) können geradkettig oder verzweigt sein und 1 bis 20, bevorzugt 1 bis 12, besonders bevorzugt 1 bis 6, Kohlenstoffatome haben, beispielsweise Methyl, Äthyl, Propyl, Isopropyl, Butyl, Isobutyl, Hexyl, Decyl, Hexadecyl oder Eikosyl.

Bevorzugte Alkylreste ($R^2$,$R^3$) sind beispielsweise Methyl, Äthyl, Propyl, Butyl, Hexyl oder Dodecyl.

Cycloalkylreste ($R^2$,$R^3$) können 5 bis 8 Ringglieder haben, beispielsweise Cyclopentyl, Cyclohexyl, Cycloheptyl oder Cyclooctyl.

BAD ORIGINAL

Aralkylreste ($R^2$,$R^3$) sind solche mit 1 bis 4 Kohlenstoffatomen im aliphatischen Teil und 6 bis 10 Kohlenstoffatomen im aromatischen Teil, beispielsweise Benzyl, ß-Phenyl-äthyl oder α- oder ß-Naphthyl-methyl.

Arylreste ($R^2$,$R^3$) sind carbocyclische, aromatische Ringsysteme mit 6 bis 10 Kohlenstoffatomen, beispielsweise Phenyl oder Naphthyl.

Selbstverständlich können alle genannten Reste ($R^1$,$R^2$,$R^3$) Substituenten tragen, die unter den Reaktionsbedingungen inert sind. Als solche Substituenten kommen beispielsweise in Frage: $C_1$-$C_4$-Alkyl, $C_1$-$C_4$-Alkoxy, Amino, Hydroxy oder eine weitere Formamidgruppe.

Für den Fall, daß die Reste $R^2$ und $R^3$ gemeinsam mit dem Stickstoffatom und gegebenenfalls mit weiteren Heteroatomen einen 5- bis 8-gliedrigen heterocyclischen Ring bilden, seien als weitere Heteroatome Sauerstoff, Schwefel oder ein zweites Stickstoffatom genannt. Solche heterocyclischen Ringsysteme sind beispielsweise: Pyrrolidin, Piperidin, Hexamethylenimin, Morpholin oder Thiomorpholin.

Formamide für das erfindungsgemäße Verfahren sind bekannt und können beispielsweise mit Hilfe der Graf-Ritter-Reaktion aus Cyanwasserstoff und Olefinen, Alkoholen, Estern oder anderen Verbindungen, die in stark saurem Medium Carboniumionen bilden, hergestellt werden (DT-OS 2 144 230). Analog können auch Bisformamide, wie beispielsweise die Reaktionsprodukte aus Dicyclopentadien oder Limonen mit

BAD ORIGINAL

wäßrigen Säuren und Cyanwasserstoff, erfindungsgemäß zu den entsprechenden Diaminen umgesetzt werden.

Im erfindungsgemäßen Verfahren können auch Formamide eingesetzt werden, die durch Formylierung von Aminen hergestellt werden (Houben Weyl, Band XI/2, Seite 27, Georg Thieme Verlag Stuttgart 1958).

Bevorzugt ist der Einsatz von Formamiden aus der Graf-Ritter-Reaktion.

Als Formamide seien beispielsweise genannt: N-Isopropylformamid, N-sek.-Butylformamid, N-1-Methylbutylformamid, N-tert.-Butylformamid, N-1-Methylhexylformamid, N-tert.-Octylformamid, N-Isodecylformamid und N-Isododecylformamid.

Besonders bevorzugt ist der Einsatz von N-tert.-Butylformamid.

Die erfindungsgemäß einsetzbaren Amine und ihre Herstellungsmethoden sind bekannt (Hoben Weiyl, Band XI/1, Georg Thieme Verlag Stuttgart 1957).

Als Amine für das erfindungsgemäße Verfahren seien beispielsweise genannt: Dimethylamin, Diäthylamin, Di-n-Butylamin, Cyclohexylamin, n-Octylamin, Dodecylamin, Octadecylamin, Hexamethylendiamin, Anilin, ortho-, metha-, para-Toluidin, 2,4- und 2,6-Diaminotoluol, 4,4'-Diaminodiphenylmethan und Diphenylamin.

BAD ORIGINAL

Das erfindungsgemäße Verfahren kann am Beispiel der Herstellung von tert.-Butylamin aus tert.-Butylformamid und Anilin durch die folgende Gleichung erläutert werden:

$$(CH_3)_3C\text{-}NH\text{-}CHO + C_6H_5\text{-}NH_2 \longrightarrow (CH_3)_3C\text{-}NH_2 + C_6H_5\text{-}NH\text{-}CHO$$

Im allgemeinen kann das erfindungsgemäße Verfahren im Temperaturbereich von etwa 75 bis etwa 250°C, bevorzugt von etwa 100 bis etwa 200°C, durchgeführt werden.

Im erfindungsgemäßen Verfahren wird die destillative Entfernung des niedriger siedenen Anteils der Umsetzungsprodukte aus dem Reaktionsgemisch durchgeführt. Dies kann beispielsweise in den nachstehend beschriebenen bevorzugten Ausführungsformen erfolgen:

a) Es wird ein Amin (II) eingesetzt, das höher als das erfindungsgemäß entstehende Amin siedet. Hierzu werden die beiden Ausgangsstoffe, nämlich das Formamid (I) und das Amin (II) in einer Destillationsanlage auf die gewünschte Reaktionstemperatur erhitzt und das erfindungsgemäß gebildete Amin durch fraktionierte Destillation am Kopf der Kolonne abgetrennt.

b) Es wird ein Amin (II) eingesetzt, dessen Formamid niedriger siedet als das ursprünglich eingesetzte Formamid (I) und als das erfindungsgemäß gebildete Amin. In diesem Falle wird das erfindungsgemäß entstehende Formamid durch fraktionierte Destillation am Kopf der Kolonne abgetrennt.

BAD ORIGINAL

c) Beide Umsetzungsprodukte haben etwa den gleichen Siedepunkt, der niedriger ist als der des eingesetzten Formamids (I) und des eingesetzten Amins (II). In diesem Falle wird das hochsiedende Formamid (I) in einer Destillationsapparatur auf Reaktionstemperatur erhitzt, das Amin (II) zudosiert und die entstehenden Umsetzungsprodukte durch fraktionierte Destillation am Kopf der Kolonne abgetrennt.

Es ist bekannt, daß bei der Hydrolyse von Formamiden in saurer Lösung sowie bei der anschließenden Isolierung der entstandenen Amine durch Zusatz von alkalisch reagierenden Stoffen (DT-PS 870 856) große Mengen von Abfallsalz-Lösungen entstehen.
Es ist weiter bekannt, daß bei der Umsetzung von wasserfreien Formamiden mit wasserfreien Alkalihydroxiden nur schwierig zu rührende Reaktionsansätze entstehen. Diese Schwierigkeit kann durch den Zusatz von Mineralöl als Rührhilfsmittel behoben werden (DT-AS 1 059 468, Beispiel 1), wobei besonders schwierig zu beseitigende Gemische von Salzen mit organischen Flüssigkeiten anfallen.
Das erfindungsgemäße Verfahren vermeidet diese Nachteile.

Im erfindungsgemäßen Verfahren werden in einer einfachen Weise die gewünschten Amine und auch die gleichzeitig entstehenden Formamide in hoher Ausbeute und in hoher Reinheit erhalten.

Es muß als ausgesprochen überraschend angesehen werden, daß das erfindungsgemäße Verfahren in einfacher Form und ohne die Verwendung eines Katalysators durchgeführt werden kann. Es ist ebenfalls überraschend, daß das erfindungsgemäße Verfahren auch auf Formamide als Einsatzstoffe ausgedehnt werden kann, deren Stickstoffatom durch verzweigte Reste sub-

BAD ORIGINAL

stituiert ist und die daher eine geringere Reaktivität aufweisen.

Die erfindungsgemäß herstellbaren Amine und Diamine sind Zwischenprodukte zur Herstellung von Herbiziden, Kautschukhilfsmitteln, Polyamiden und Polyurethanen.

BAD ORIGINAL

Beispiel 1

Eine Mischung von 101 g tert.-Butylformamid und 93 g Anilin wird in einem 300 ml-Kolben an einer Füllkörperkolonne, versehen mit regelbarem Kolonnenkopf, ansteigend auf eine Sumpftemperatur von 100 bis 200°C erhitzt, wobei im Laufe von 5 Stunden 69,4 g (95 % der Theorie) tert.-Butylamin (Reinheit nach gaschromatographischer Analyse 99,8 %) abdestillieren. Nach einem Zwischenlauf, der aus tert.-Butylamin, wenig tert.-Butylformamid, Anilin und Formanilid besteht, erhält man durch weitere Destillation im Vakuum 111 g (92 % der Theorie) Formanilid vom Siedepunkt 105°C/0,2 mbar.

Beispiel 2

In 77 g auf 150 bis 155°C erhitztes tert.-Butylformamid werden unter Rühren der Reaktionsmischung 40 g Dimethylamin eingeleitet. Dabei destillieren über eine Kolonne 58 g einer Fraktion vom Siedebereich 43 bis 37°C ab, die nach gaschromatographischer Analyse 6 % Dimethylamin und 94 % tert.-Butylamin enthält; im Sumpf bleibt in nahezu quantitativer Ausbeute reines Diemthylformamid zurück.

BAD ORIGINAL

Patentansprüche

1) Verfahren zur Herstellung von Aminen aus Formamiden, dadurch gekennzeichnet, daß man ein Formamid der Formel

$$R^1\text{-NH-CHO} \quad ,$$

in der
$R^1$ Alkyl, Cycloalkyl oder Aralkyl bedeutet,

mit einem Amin der Formel

$$R^2R^3NH \quad ,$$

in der
$R^2$ und $R^3$ Wasserstoff, Alkyl, Cycloalkyl, Aralkyl oder Aryl bedeuten,
oder in der die Reste $R^2$ und $R^3$ gemeinsam mit dem Stickstoffatom und gegebenenfalls weiteren Heteroatomen einen 5- bis 8-gliedrigen heterocyclischen Ring bilden,
wobei nur einer der Reste $R^2$ und $R^3$ Wasserstoff sein kann und
wobei wenigstens einer der Reste $R^2$ und $R^3$ verschieden von $R^1$ ist,

bei erhöhter Temperatur umsetzt und den niedriger siedenden Anteil der Umsetzungsprodukte destillativ aus dem Reaktionsgemisch entfernt.

2) Verfahren nach Anspruch 1, dadurch gekennzeichnet, daß man N-tert.-Butyl-formamid einsetzt.

3) Verfahren nach Anspruch 1 bis 2, dadurch gekennzeichnet, daß man im Temperaturbereich von etwa 100 bis etwa 250°C arbeitet.



BAD ORIGINAL

4) Verfahren nach Anspruch 1 bis 3, dadurch gekennzeichnet, daß man ein Amin einsetzt, das höher als das entstehende Amin siedet und das entstehende Amin destillativ aus dem Reaktionsgemisch entfernt.

5) Verfahren nach Anspruch 1 bis 3, dadurch gekennzeichnet, daß man ein Amin einsetzt, dessen Formamid niedriger siedet als das eingesetzte Formamid und das entstehende Formamid destillativ aus dem Reaktionsgemisch entfernt.

6) Verfahren nach Anspruch 1 bis 3, dadurch gekennzeichnet, daß man bei einem erfindungsgemäß entstehenden Amin und einem erfindungsgemäß entstehenden Formamid, deren Siedepunkte eng beieinander liegen und niedriger sind als die der Ausgangsstoffe, das entstehende Amin und das entstehende Formamid gemeinsam destillativ aus dem Reaktionsgemisch entfernt.

BAD ORIGINAL

BAD ORIGINAL

0001798

Europäisches Patentamt

# EUROPÄISCHER RECHERCHENBERICHT

Nummer der Anmeldung

EP 78 101 207.5

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | | | C 07 C 85/20 |
| - | HOUBEN-WEYL "Methoden der organischen Chemie", Band XI/1, 1957, Thieme Verlag, Stuttgart, Seite 939<br>-- | 1 | |
| - | HOUBEN-WEYL "Methoden der organischen Chemie", Band XI/2, 1958, Thieme Verlag, Stuttgart, Seite 29, Zeilen 15 bis 26<br>-- | 1 | RECHERCHIERTE SACHGEBIETE (Int. Cl.²) |
| A | DE - C - 81 539 CHEMISCHE FABRIK)<br>---- | | C 07 C 85/00<br>C 07 C 85/20<br>C 07 C 102/00<br>C 07 C 103/34 |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 07-02-1979 | KAPTEYN |